# EUROPEAN PATENT SPECIFICATION

(11) **EP 2 402 363 B1**
(45) Date of publication and mention of the grant of the patent: **16.04.2014**
(21) Application number: 10746471.1
(22) Date of filing: 26.02.2010
(51) Int. Cl.: C07K 1/02, C12P 13/12, C07C 323/58

(54) **METHOD FOR INCREASING THE SOLUBILITY OF METHIONINE BY MINERAL ADDITION AND ACID TREATMENT**
VERFAHREN ZUR ERHÖHUNG DER LÖSLICHKEIT VON METHIONIN MITTELS MINERALZUGABE UND SÄUREBEHANDLUNG
PROCÉDÉ POUR AUGMENTER LA SOLUBILITÉ DE LA MÉTHIONINE PAR AJOUT DE MINÉRAUX ET TRAITEMENT ACIDE

(30) Priority: 27.02.2009 KR 20090016605
(43) Date of publication of application: 04.01.2012
(73) Proprietor: CJ CheilJedang Corporation, Seoul, 100-400 (KR)
(72) Inventor: KIM, So Young, Gwacheon-si Gyeonggi-do 427-736 (KR); SHIN, Yong Uk, Yongin-si Gyeonggi-do 448-972 (KR); HEO, In Kyung, Seoul 157-031 (KR); KIM, Hyun Ah, Namwon-si Jeollabuk-do 590-972 (KR); KIM, Ju Eun, Seoul 157-200 (KR); SEO, Chang Il, Incheon 401-030 (KR); SON, Sung Kwang, Seoul 139-925 (KR); LEE, Sang Mok, Seoul 157-931 (KR); JHON, Sung Hoo, Seoul 157-200 (KR); LEE, Han Jin, Seoul 136-767 (KR); NA, Kwang Ho, Seoul 157-201 (KR); KIM, Il Chul, Seongnam-si Gyeonggi-do 463-725 (KR)
(74) Representative: Forrest, Graham Robert
(86) International application number: PCT/KR2010/001254
(87) International publication number: WO 2010/098632

(56) References cited:
- EP-A2- 0 556 498
- EP-A2- 1 976 399
- WO-A1-96/11907
- KR-A- 20050 110 167
- KR-B1- 880 002 033
- US-A- 4 764 633
- US-A1- 2005 089 975

## Description

### BACKGROUND OF THE INVENTION

### 1. Field of the Invention

The present invention relates to a method for increasing the solubility of methionine.

### 2. Description of the Related Art

Methionine is one of the essential amino acids in the body, and has been widely used as an animal feed and food additive, as well as a component of medical aqueous solutions and other raw material for medicinal products. Methionine acts as a precursor of choline (lecithin) and creatine, and is also used as a raw material for the synthesis of cysteine and taurine. In addition, it functions as a sulfur donor. S-adenosyl-methionine is derived from L-methionine and serves as a methyl donor in the body, and it is involved in the synthesis of various neurotransmitters in the brain. Methionine and/or S-adenosyl-L-methionine (SAM) is/are also found to prevent lipid accumulation in the liver and arteries and to be effective for the treatment of depression, inflammation, liver diseases and muscle pain (Jeon BR et al., J Hepatol., 2001 Mar; 34(3): 395-401).

For the chemical synthesis of methionine, L-methionine is produced through the hydrolysis of 5-(β-methylmercaptoethyl)-hydantoin. However, the chemically synthesized methionine is disadvantageously present in a mixture of L- and D-forms. Therefore, the present inventors developed a biological method for selectively synthesizing L-methionine, and they have already applied for a patent (WO 2008/103432). The method, termed in brief "a two-step process", comprises the fermentative production of an L-methionine precursor and the enzymatic conversion of the L-methionine precursor to L-methionine. The L-methionine precursor preferably includes O-acetyl homoserine and O-succinyl homoserine. Also, compared to the conventional chemical synthesis of producing DL-methionine simultaneously, the two-step process has the advantage of being selective for L-methionine only, with the concomitant production of organic acid, more particularly, succinic acid or acetic acid as a useful by-product. L-methionine obtained in the two-step process is included in a microorganism-fermented solution during the precursor production process, and generally exists as an aqueous solution form.

In this regard, the solubility of DL-methionine or L-methionine in the aqueous solution is generally about 5% (w/v). When methionine is used in the aqueous solution form, preparation of high concentration of methionine aqueous solution is occasionally needed. Disadvantageously, it is difficult to prepare the high concentration of methionine aqueous solution, because of the low solubility of methionine. The high concentration of methionine aqueous solution can be directly used in feed or the like, and its volume is less than low concentration thereof. In addition, the high concentration of methionine aqueous solution can be easily used in various applications such as formulation modification and preparation of derivatives.

As the conventional methods for increasing the methionine solubility, the use of mineral or acid treatment is disclosed. US Patent No. 5430164 discloses the use of mineral to increase the solubility of DL-methionine up to 12% (w/v). In a paper, Dominik Fuchs et al. reported that acid treatment is used to increase the solubility of DL-methionine to 18% (w/v) particularly in pH 2 (Dominik Fuchs, et al., Ind. Eng. Chem. Res. 2006, 45, 6578-6584).

US 2005/0089975 also discloses methods of increasing methionine solubility by pH adjustment.

US Patent No. 5430164 discloses a method of increasing the solubility by chelation of DL-methionine using minerals. The chelation of DL-methionine is able to increase the solubility of L-methionine by formation of a 1:1 or 1:2 chelate complex of mineral and DL-methionine. However, this method is disadvantageous in that the high content of mineral is also required for the solubilization of high concentration of DL-methionine. In particular, total 3.5 to 3.9 M (39.6% to 49.6% of total methionine solution) of minerals, including 3.35 M zinc sulfate and 0.167 to 0.569 M ferric chloride, are required for the preparation of 1 L of maximum 50% methionine solution. The use of relatively expensive minerals increases costs of raw materials. Hence, the amount of minerals should be reduced to obtain economic benefits in the mass production for industrialization. Meanwhile, the method of increasing the solubility of DL-methionine by acid treatment has a problem of strong acidification of products.

To solve the above problems, the present inventors therefore demonstrated that the methionine solubility can be maximized to 50% by the combination of mineral addition and acid addition and in this case the amount of mineral is only less than 15% compared to the method used before, thereby completing the present invention.

### SUMMARY OF THE INVENTION

It is an object of the present invention to provide a method for maximizing the methionine solubility with minimal use of minerals, and to provide a methionine solution having increased methionine solubility according to the method.

### Effect of the invention

The method of the present invention is able to prepare high concentration of methionine solution with minimal use of various minerals. Thus, the high concentration of methionine solution can be directly used in feed or the like, and its volume is less than low concentration thereof. In addition, the high concentration of methionine solution can be easily used in various applications such as formulation modification and preparation of derivatives.

### BRIEF DESCRIPTION OF THE DRAWINGS

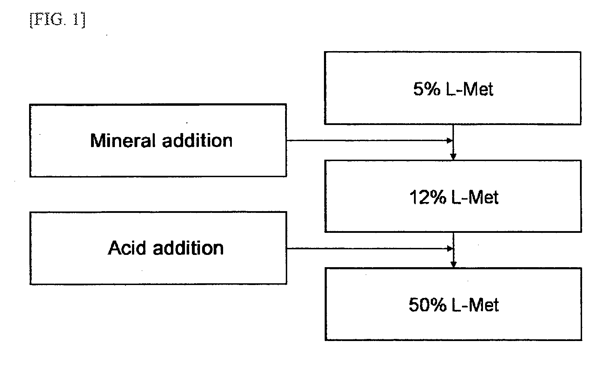

FIG. 1 is a flow chart showing a process for improving the solubility of L-methionine to 50% according to the present invention.

### DETAILED DESCRIPTION OF THE PREFERRED EMBODIMENTS

In order to achieve the above object, an aspect of the present invention is to provide a method for increasing the solubility of methionine solution, comprising
step 1 of adding a mineral containing one or more bivalent metal ions to a methionine-containing solution; and
step 2 of adding acid to the methionine-containing solution obtained in step 1.

Another aspect of the present invention is to provide a methionine solution having methionine solubility from 35% to 50%, prepared by the above method.

Hereinafter, the constitution of the present invention will be described in detail.

An aspect of the present invention is to provide a method for increasing the solubility of methionine solution,
comprising :
step 1 of adding a mineral containing one or more bivalent metal ions to a methionine-containing solution; and
step 2 of adding acid to the methionine-containing solution obtained in step 1.

The present invention is technically characterized in that the use of metal ions for the preparation of high concentration of methionine solution is greatly reduced by combination of chelation using bivalent metal ions and pH drop by acid treatment, as compared to the conventional methods.

In one specific embodiment of the present invention, the methionine-containing solution may be a solution containing DL-methionine or L-methionine. In another specific embodiment of the present invention, the methionine-containing solution may be a solution containing L-methionine produced by fermentation or enzymatic conversion, or L-methionine concentrate prepared by its purification, concentration or dry process, or a L-methionine solution made by re-solubilization of L-methionine dried-powder. In this regard, the purity of L-methionine may be 10% to 100% In the specific embodiment of the present invention, L-methionine was prepared by the fermentation of a microorganism strain, and the enzymatic conversion reaction by the method as described in WO2008/013432. In the specific embodiment of the present invention, L-methionine was prepared by the enzymatic conversion reaction after the fermentation, followed by the recovery using a fluid-bed granulator in a dry form of powdery granule. The L-methionine in a form of powdery granule has a purity of approximately 60%

In the L-methionine-containing solution produced by the fermentation and enzymatic conversion reaction, the solubility of L-methionine may be increased up to 35% according to the present invention. It is suggested that the L-methionine-containing solution produced by the fermentation and enzymatic conversion reaction has the high content of impurities except L-methionine, and thus it has the lower solubility than that of methionine with the purity of 90% or more. Therefore, as the purity of methionine is increased, the solubility of methionine can be increased from 35% to 50%. In the specific embodiment of the present invention, in the case of using L-methionine with the purity of 99% or more that is prepared by purification of the L-methionine produced by the fermentation and enzymatic conversion reaction, the experimental results were found to be identical to those of commercially available L-methionine with the purity of 99%, which was purchased from Sigma.

In the present invention, the bivalent metal ion may be selected from the group consisting of Fe²⁺, Ca²⁺, Mg²⁺, Mn²⁺, Cu²⁺ and Zn²⁺, and preferably selected from the group consisting of Fe²⁺, M²⁺, and Zn²⁺.

The mineral containing bivalent metal ions may be one or more selected from the group consisting of iron sulfide, manganese sulfide and zinc sulfide, and more preferably minerals having a lower molecular weight. The minerals may be used alone or in a mixture of two or more thereof. More preferably, it is avoided to use minerals having a higher molecular weight or expensive minerals. The amount of minerals may be preferably used in a concentration of 1 to 10%, and more preferably 2 to 8%, and most preferably 3 to 6%, based on the total volume of methionine solution.

In the present invention, the acid treatment may be preferably performed by adding an acid at a concentration of 0.01 to 0.5 M to the methionine-containing solution, more preferably 0.05 to 0.4 M, and most preferably 0.1 M In the specific Example, 0.1 M was used. In the present invention, the acid may be sulfuric acid, but is not limited thereto.

In the specific Example of the present invention, the mineral from 1.88% up to 8.43% was used with respect to methionine with various purities, thereby preparing a methionine solution of 35 to 50% (Table 1). In addition, total injection amounts of the minerals were more reduced by using the mixture of two or more of the minerals, as compared to the single use of the minerals (see Table 1). According to the present invention, the general solubility of methionine in water can be maximized to 10 times or higher by the acid treatment, following the addition of a minimal amount of minerals for the improvement of methionine solubility.

Another aspect of the present invention is to provide a high concentration of methionine solution having increased methionine solubility according to the above method.

The methionine solution may be purified by an additional purification process, and then prepared in a form of dry powder or in a form of solution prepared by solubilizing it in an aqueous solution.

The acid treatment following the mineral addition according to the present invention is able to critically increase the solubility of methionine without causing the problems of using an excessive amount of minerals and strong acidification of products due to acid addition, compared to the conventional methods in which mineral addition or acid addition is separately performed. Thus, the produced methionine solution can be applied to various fields including animal feeds, food additives, medicines, and other raw materials for medicinal products.

Hereinafter, the constitutions and effects of the present invention will be described in more detail with reference to Examples. However, these Examples are for illustrative purposes only, and the invention is not intended to be limited by these Examples.

### Example 1: Preparation of 50% Methionine solution by Acid addition After Preparation of 12% Methionine solution using Manganese sulfide

Generally, the solubility of methionine in water is known to be approximately 50∼55 g/L at room temperature. Therefore, 2.5 g of L-methionine (99%, Sigma, USA) was dissolved in 50 mL of water to prepare a 5% L-methionine solution (50 g/L). While this solution was stirred at 70∼80°C, 23.5 mM L-methionine (3.5 g) and 23.5 mM manganese sulfide (3.97 g) were added so as to prepare a 12% L-methionine solution (120 g/L). The amount of manganese sulfide is calculated to be a molar ratio of additional L-methionine and manganese sulfide should be 1:1 After complete dissolving of L-methionine crystals, an additional 40g of L-methionine (about 40 g) was added to the 12% L-methionine solution at room temperature under stirring. During this process, 98% sulfuric acid (36.8 N) was added to the L-methionine solution to achieve a final concentration of 0.1 M, leading to exothermic reaction. After termination of the exothermic reaction, filtration was performed to remove the residual L-methionine crystal that was not dissolved. After the filtration, L-methionine concentration in the L-methionine solution was determined by HPLC, And the L-methionine concentration was 50% (500 g/L) in the solution. 50 ml of the initial 5% methionine solution was finally increased to become 80 ml of 50% methionine solution by the addition of the excessive amount of methionine during the process of increasing the methionine solubility. Therefore, 80 ml of 50% methionine solution was finally obtained.

The same experiment, was performed using DL-methionine (99%, Sigma, USA), so as to obtain the identical results.

### Example 2: Preparation of 50% Methionin solution by Acid addition After Preparation of 12% Methionine solution using Zinc sulfide

After preparation of 5% L-methionine solution as in Example 1, 23.5 mM L-methionine (3.5 g) was added to 50 ml thereof under stirring at 70∼80°C. Then, 23.5 mM zinc sulfide (6.75 g) was injected to be a molar ratio of additional L-methionine and zinc sulfide of 1:1, so as to prepare a 12% L-methionine solution (120 g/L). After completely dissolving L-methionine crystals, an excessive amount of L-methionine (about 40 g) was added to the 12% L-methionine solution at room temperature under stirring. During this process, 98% sulfuric acid (36.8 N) was added to the L-methionine solution to be 0.1 M, leading to exothermic reaction. After termination of the exothermic reaction, filtration was performed to remove the residual L-methionine crystals that were not dissolved, and the concentration of L-methionine was measured. The L-methionine solution was a 50% (500 g/L) solution. The concentration of L-methionine was determined by HPLC.

50 ml of the initial 5% methionine solution was finally increased to become 80 ml of 50% methionine solution by addition of the excessive amount of methionine during the process of increasing the methionine solubility. Therefore, 80 ml of 50% methionine solution was finally obtained.

The same experiment was performed using DL-methionine, so as to obtain the identical results.

### Example 3: Preparation of solution of 50% Methionine and 3.75% Minerals by Acid addition After Preparation of 12% Methionine solution using Manganese sulfide, Zinc sulfide, and Iron sulfide

After preparation of 5% L-methionine solution as in Example 1, 23.5 mM L-methionine (3.5 g) was added to 50 ml thereof under stirring at 70∼80°C. Additionally, each 1 g of manganese sulfide, zinc sulfide, and iron sulfide was added, so as to prepare a 12% L-methionine solution (120 g/L). In this process, each of the minerals became 5.92 mM manganese sulfide, 3.48 mM zinc sulfide, and 2.5 mM iron sulfide. After completely dissolving L-methionine crystals, an excessive amount of L-methionine (about 40 g) was added to the 12% L-methionine solution at room temperature under stirring. During this process, 98% sulfuric acid (36.8 N) was added to the L-methionine solution to be 0.1 M, leading to exothermic reaction. After termination of the exothermic reaction, filtration was performed to remove the residual L-methionine crystals that were not dissolved, and the concentration of L-methionine was measured. The solution was a solution of 50% (500 g/L) L-methionine and 3.75% minerals. The concentration of L-methionine was determined by HPLC.

50 ml of the initial 5% methionine solution was finally increased to become 80 ml of 50% methionine solution by addition of the excessive amount of methionine during the process of increasing the methionine solubility. Therefore, 80 ml of 50% methionine solution was finally obtained.

The same experiment was performed using DL-methionine, so as to obtain the identical results.

### Example 4: Preparation of solution of 50% Méthionine and 1.88% Minerals by Acid addition After Preparation of 12% Methionine solution using Manganese sulfide, Zinc sulfide, and Iron sulfide

After preparation of 5% L-methionine solution as in Example 2, 23.5 mM L-methionine (3.5 g) was added to 50 ml thereof under stirring at 70∼80°C. Additionally, each 0.5 g of manganese sulfide, zinc sulfide, and iron sulfide was added, so as to prepare a 12% L-methionine solution (120 g/L). In this process, each of the minerals became 2.96 mM manganese sulfide, 1.74 mM zinc sulfide, and 1.25 mM iron sulfide. After completely dissolving L-methionine crystals, an excessive amount of L-methionine (about 40 g) was added to the 12% L-methionine solution at room temperature under stirring. During this process, 98% sulfuric acid (36.8 N) was added to the L-methionine solution to be 0.1 M, leading to exothermic reaction. After termination of the exothermic reaction, filtration was performed to remove the residual L-methionine crystals that were not dissolved, and the concentration of L-methionine was measured. The solution was a solution of 50% (500 g/L) L-methionine and 1.88% minerals. The concentration of L-methionine was determined by HPLC.

50 ml of the initial 5% methionine solution was finally increased to become 80 ml of 50% methionine solution by addition of the excessive amount of methionine during the process of increasing the methionine solubility. Therefore, 80 ml of 50% methionine solution was finally obtained.

The same experiment was performed using DL-methionine, so as to obtain the identical results.

### Example 5: Preparation of L-Methionine solution using L-Methionine produced by Fermentation and Conversion reaction

In the present Example, L-methionine powder that was prepared by enzymatic conversion reaction of O-acetyl homoserine produced by fermentation was used to prepare high concentration of L-methionine.

First, an L-methionine solution was prepared by enzymatic conversion reaction of O-acetyl homoserine produced by fermentation, and then this solution was dried to prepare L-methionine granules using a fluid-bed granulator. The content of L-methionine in the L-methionine granules was quantified by HPLC, and its purity was found to be approximately 60%. The specific preparation method of the L-methionine solution is described in the prior art, WO2008/013432.

The L-methionine granules were quantified to prepare a 5% L-methionine solution 5.83 g of L-methionine granules were added to 50 ml thereof under stirring at 70∼80°C. Additionally, 5 g of iron sulfide (13 mM) was added, so as to prepare a 12% L-methionine solution (120 g/L). After completely dissolving L-methionine granules, an excessive amount of L-methionine (about 67 g) was added to the 12% L-methionine solution at room temperature under stirring. During this process, 98% sulfuric acid (36.8 N) was added to the solution to be 0.1 M, leading to exothermic reaction. After termination of the exothermic reaction, filtration was performed to remove the residual L-methionine granule crystals that were not dissolved, and the concentration of L-methionine was measured The solution was 80 ml of 35% L-methionine solution (350 g/L). The concentration of L-methionine was determined by HPLC. It is suggested that the concentration of this solution is lower than those of L-methionine or DL-methionine with the purity of 99% in the above Examples, because 40% impurities present in the L-methionine granules are also dissolved in the solution so as to inhibit solubilization of L-methionine.

To confirm this, L-methionine with the purity of 99% or more was prepared from the L-methionine solution produced by fermentation and enzymatic conversion. The L-methionine solution was titrated to pH 1.0 with sulfuric acid, and adsorbed onto cation exchange resin, and eluted with an ammonia solution. The eluent was titrated to pH 7.0 with sulfuric acid, and then heated to prepare a 2X concentrated solution. An excessive amount of methanol was added to the concentrated solution to induce crystallization, and the formed crystals were recovered and dried. The content of the dried crystals was quantified by HPLC. The crystals were found to have the purity of 99% or more. Using the recovered L-methionine crystal powder, experiments were performed in the same manner as in Examples 1 to 4. At a result, a methionine solution of 50% or higher can be prepared as in the above Examples.

### Example 6: Comparison of mineral amounts used in Examples 1 to 5

Manganese sulfide having a molecular weight of 169.02 g/mole, zinc sulfide having a molecular weight of 287.53 g/mole, and iron sulfide having a molecular weight of 399,88 g/mole were used, and total amounts of the minerals used in Examples 1 to 5 were compared.

**[Table 1]**

| | Methionine | | | Manganese sulfide | | Zinc sulfide | | Iron sulfide | | Final miner als |
|---|---|---|---|---|---|---|---|---|---|---|
| | g | mole | wt% | mole | g | mole | g | mole | g | wt% |
| Ex.1 | 40 | 0.268 | 500 | 0.0235 | 3.97 | | | | | 4.96 |
| Ex.2 | 40 | 0.268 | 500 | | | 0.023 | 6.75 | | | 8.43 |
| Ex.3 | 40 | 0.268 | 500 | 0.0059 | 1.00 | 0.0035 | 1.00 | 0.0025 | 1.00 | 3.75 |
| Ex.4 | 40 | 0.268 | 500 | 0.0030 | 0.50 | 0.0017 | 0.50 | 0.0013 | 0.50 | 1.88 |
| Ex.5* | 28 | 0.188 | 350 | | | | | 0.0125 | 5.00 | 6.25 |

| | | | | | | | | | | |
|---|---|---|---|---|---|---|---|---|---|---|
| * Use of methionine powder produced by fermentation and enzymatic conversion - Final volume of solution is 80 ml - Conc. of sulfuric acid 0.1 mole/L in final solution | | | | | | | | | | |

As shown in Table 1, the final amount of minerals added to prepare 1 L of 35 to 50% methionine solutions was within 1 to 10%, indicating that a very small amount thereof was required. In addition, it was found that when two or more of the minerals were used, their addition amounts were remarkably reduced compared to the single use of the minerals.

Taken together, the addition amounts of minerals were greatly reduced, as compared to the use of 40 to 50% minerals for the preparation of 8 to 50% methionine solutions, described in US Patent No. 5430164. Therefore, the method of the present invention can be used for the preparation of high concentration of methionine solution with improved methionine solubility while remarkably reducing the use of minerals.

### Industrial Applicability

As described in the above Examples, the present invention provides a method for maximizing the general solubility of methionine in water to 10 times or higher by the acid treatment, following the addition of a minimal amount of minerals to a methionine-containing solution for the improvement of methionine solubility.

## Claims

1. A method for increasing the solubility of methionine to produce methionine solution, comprising:
step 1 of adding a mineral containing one or more bivalent metal ions to a methionine-containing solution; and
step 2 of adding acid to the methionine-containing solution obtained in step 1.

2. The method according to claim 1, wherein the bivalent metal ion is one or more selected from the group consisting of Fe²⁺, Ca²⁺, Mg²⁺, Mn²⁺, Cu²⁺ and Zn²⁺.

3. The method according to claim 1, wherein the mineral containing bivalent metal ion is one or more selected from the group consisting of iron sulfide, manganese sulfide, and zinc sulfide.

4. The method according to claim 1, wherein the acid is sulfuric acid.

5. The method according to claim 1, wherein the methionine is DL-methionine or L-methionine.

6. The method according to claim 1, wherein the methionine-containing solution is a solution containing L-methionine produced by fermentation or enzymatic conversion.

7. The method according to claim 1, wherein the amount of mineral added to the solution is to achieve the concentration of 1 to 10% based on the total volume of the methionine-containing solution.

8. The method according to claim 1, wherein the amount of acid added to the methionine-containing solution is to achieve the concentration of 0.01 to 0.5 M.

9. A methionine solution prepared by a method according to any one of claims 1 to 8, wherein the solubility of methionine is from 35% to 50%.

## Patentansprüche

1. Verfahren zur Erhöhung der Löslichkeit von Methionin zur Herstellung einer Methioninlösung, das Folgendes umfasst:
Schritt 1 des Zusetzens eines Minerals, das ein oder mehrere zweiwertige Metallionen umfasst, zu einer methioninhältigen Lösung; und
Schritt 2 des Zusetzens von Säure zu der in Schritt 1 erhaltenen methioninhältigen Lösung.

2. Verfahren nach Anspruch 1, wobei das zweiwertige Metallion ein oder mehrere aus der aus Fe²⁺, Ca²⁺, Mg²⁺, Mn²⁺, Cu²⁺ und Zn²⁺ bestehenden Gruppe ausgewähltes ist.

3. Verfahren nach Anspruch 1, wobei das Mineral, das zweiwertige Metallionen enthält, ein oder mehrere aus der aus Eisensulfid, Mangansulfid und Zinksulfid bestehenden Gruppe ausgewähltes ist.

4. Verfahren nach Anspruch 1, wobei die Säure Schwefelsäure ist.

5. Verfahren nach Anspruch 1, wobei das Methionin DL-Methionin oder L-Methionin ist.

6. Verfahren nach Anspruch 1, wobei die methioninhältige Lösung eine Lösung ist, die L-Methionin enthält und durch Fermentation oder enzymatische Überführung hergestellt wird.

7. Verfahren nach Anspruch 1, wobei die Mineralmenge, die zur Lösung zugesetzt wird, so gewählt wird, das eine Konzentration von 1 bis 10 %, bezogen auf das Gesamtvolumen der methioninhältigen Lösung, erhalten wird.

8. Verfahren nach Anspruch 1, wobei die Säuremenge, die zur methioninhältigen Lösung zugesetzt wird, so gewählt wird, dass eine Konzentration von 0,01 bis 0,5 M erhalten wird.

9. Methioninlösung, die durch ein Verfahren nach einem der Ansprüche 1 bis 8 hergestellt wurde, wobei die Löslichkeit von Methionin 35 % bis 50 % beträgt.

## Revendications

1. Méthode pour augmenter la solubilité de la méthionine pour produire une solution de méthionine, comprenant :
l'étape 1 consistant à ajouter un minerai contenant un ou plusieurs ions métalliques bivalents à une solution contenant de la méthionine, et
l'étape 2 consistant à ajouter un acide à la solution contenant de la méthionine obtenue à l'étape 1.

2. Méthode selon la revendication 1, dans laquelle le ion métallique bivalent est un ou plusieurs sélectionnés dans le groupe consistant en Fe²⁺, Ca²⁺, Mg²⁺, Mn²⁺, Cu²⁺ et Zn²⁺.

3. Méthode selon la revendication 1, dans laquelle le minerai contenant l'ion métallique bivalent est un ou plusieurs sélectionnés dans le groupe consistant en sulfure de fer, sulfure de manganèse et sulfure de zinc.

4. Méthode selon la revendication 1, dans laquelle l'acide est l'acide sulfurique.

5. Méthode selon la revendication 1, dans laquelle la méthionine est la méthionine DL ou la méthionine L.

6. Méthode selon la revendication 1, dans laquelle la solution contenant de la méthionine est une solution contenant la méthionine L produite par fermentation ou conversion enzymatique.

7. Méthode selon la revendication 1, dans laquelle la quantité de minerai ajoutée à la solution est pour atteindre une concentration de 1 à 10 % sur la base du volume total de la solution contenant de la méthionine.

8. Méthode selon la revendication 1, dans laquelle la quantité d'acide ajouté à la solution contenant de la méthionine est pour atteindre la concentration de 0,01 à 0,5 M.

9. Solution de méthionine préparée par une méthode selon l'une quelconque des revendications 1 à 8, dans laquelle la solubilité de la méthionine est de 35 % à 50 %.
